# EUROPEAN PATENT APPLICATION

(11) **EP 4 420 529 A1**
(43) Date of publication of application: **28.08.2024**
(21) Application number: 21961328.8
(22) Date of filing: 19.10.2021
(51) Int. Cl.: A24B 13/00, A24B 15/00

(54) **ORAL COMPOSITION CONTAINING TREHALOSE**

(71) Applicant: Japan Tobacco Inc., Tokyo 105-6927 (JP)
(72) Inventor: HAMAMOTO, Takeshi, Tokyo 130-8603 (JP); FURUKOSHI, Masashi, Tokyo 130-8603 (JP)
(74) Representative: Hoffmann Eitle
(86) International application number: PCT/JP2021/038528
(87) International publication number: WO 2023/067675

(57) **Abstract**

The present invention addresses the problem of providing an oral composition in which moisture absorption is suppressed and that is excellent in storage stability. The oral composition contains nicotine and trehalose, wherein the content of trehalose is from 21 to 99 wt %.

## Description

### Technical Field

The present invention relates to an oral composition containing trehalose.

### Background Art

Oral pouch products, such as oral tobacco products, are packaged articles including a pouch (packaging material) formed from a material like nonwoven fabric and an oral composition containing, for example, a flavor source housed in it. The user uses an oral pouch product by placing it in the oral cavity.

When an oral pouch product is inserted into the oral cavity of the user, ingredients contained in the oral composition, such as nicotine, seep out through the packaging material, delivering a flavor component to the user

For oral compositions contained in oral pouch products, it is known that their storage stability can be increased by controlling bacterial growth through pH adjustment. Examples of pH-adjusting agents include alkali metal hydroxides, such as sodium hydroxide and potassium hydroxide (Patent Document 1).

In Patent Document 2, furthermore, a dry powder nicotine formulation suitable for inhalation is disclosed that includes nicotine particles generated from nicotine and sugar.

### Citation List

### Patent Document

Patent Document 1: Japanese Unexamined Patent Application Publication (Translation of PCT Application) No. 2016-536982

Patent Document 2: Japanese Unexamined Patent Application Publication (Translation of PCT Application) No. 2017-512212

### Summary of Invention

### Technical Problem

As well as through bacterial growth, an oral composition degrades through moisture absorption caused by environmental water during storage. When the oral composition undergoes moisture absorption, ingredients in the composition alter because of the water, causing a decrease in the quality of the product.

A possible way to suppress the moisture absorption is the method of improving the seal using a container, but it is unfavorable from a cost perspective. Enclosing a substance such as a desiccant, furthermore, is unfavorable because it results in an increase in product volume during storage.

The present invention, therefore, addresses the problem of providing an oral composition in which moisture absorption is suppressed and that is excellent in storage stability.

### Solution to Problem

The inventor found that the above problem can be addressed by allowing the oral composition to contain a specific amount of trehalose, and arrived at the present invention on the basis of this finding.

That is, the present invention is as follows.
[1] An oral composition containing nicotine and trehalose, wherein a content of the trehalose is from 21 to 99 wt %.
[2] The oral composition according to [1], wherein the trehalose is α,α-trehalose or α,β-trehalose.
[3] The oral composition according to [1] or [2], wherein the trehalose is α,α-trehalose.
[4] The oral composition according to any of [1] to [3], wherein the trehalose is anhydrous trehalose.
[5] The oral composition according to any of [1] to [4], wherein the trehalose is present as a crystal powder.
[6] The oral composition according to [5], wherein an average particle size of the trehalose is from 100 to 1200 µm.
[7] An oral pouch product composed of the oral composition according to any of [1] to [6] and a packaging material in which the oral composition is packaged.

### Advantageous Effects of Invention

According to the present invention, it is possible to provide an oral composition in which moisture absorption is suppressed and that is excellent in storage stability.

### Description of Embodiments

Embodiments of the present invention will now be described in detail. These descriptions, however, are examples (representative examples) of embodiments of the present invention, and the present invention is not limited to what is described unless it goes beyond the scope thereof.

A numerical range expressed using ("from" and) "to" herein indicates a range that includes the values before and after "to" as the lower and upper limits, with "(from) A to B" meaning that the value is A or more and B or less.

### [Oral Composition]

An oral composition according to an embodiment of the present invention contains nicotine and trehalose. An oral composition in this embodiment refers to a composition that is used by being inserted into the oral cavity of a user.

When an oral composition contains trehalose, the trehalose takes in water as much as up to approximately 10% of its weight, helping suppress moisture absorption by the oral composition. This hygroscopic effect is markedly strong compared with that of other saccharides. After moisture absorption, the trehalose turns into crystalline hydrous trehalose. The crystalline hydrous trehalose itself, however, is a powder; it is not sticky or flowable and does not have negative impacts on the appearance and feel during use of the product.

Moisture absorption by an oral composition, furthermore, can encourage microbial growth in it. Suppressing moisture absorption by an oral composition, therefore, contributes to limiting a decrease in the quality of the product resulting from microbial growth.

Trehalose, furthermore, is advantageous in that when added to a composition, it does not have negative impacts on the savor of the composition because it only exhibits a slight and good sweet taste.

Moreover, trehalose has no reducing properties because it is a disaccharide generated through a reaction between reducing groups of two glucoses. The trehalose itself, therefore, does not oxidize or degrade and is of high stability.

### (Nicotine)

The oral composition in this embodiment contains nicotine. The form in which nicotine is contained is not particularly restricted. For example, nicotine as a compound may be contained, or a nicotine carrier, such as a nicotine salt or stabilized nicotine (e.g., nicotine supported on an ion-exchange resin), may be contained.

An example of a nicotine carrier is, as mentioned above, a substance including an ion-exchange resin and nicotine supported on it.

When nicotine is supported on an ion-exchange resin, an ion-exchange resin is used as a support. An example of an ion-exchange resin is a weakly acidic cation-exchange resin. An ion-exchange resin with nicotine supported on it can be a resin complex that contains, for example, 10 wt % or more and 20 wt % or less nicotine, referred to as nicotine polacrilex. The ion-exchange resin used in nicotine polacrilex is a weakly acidic cation-exchange resin.

When nicotine polacrilex is used, the amount of nicotine polacrilex added is typically 0.5 wt % or more, preferably 1.0 wt % or more, more preferably 2.0 wt % or more in relation to the oral composition. For the savor of the oral composition, however, the amount of nicotine polacrilex added is typically 15.0 wt % or less, preferably 12.0 wt % or less, more preferably 10.0 wt % or less in relation to the oral composition.

As a nicotine source, furthermore, a tobacco material including a tobacco powder obtained by grinding tobacco leaves, for example, may be contained.

The tobacco powder is one that may include substances such as shreds, fine powder, and fibers of the lamina of dried tobacco leaves and that can be prepared by the method described below. Tobacco leaves herein may include the lamina, the stem, and the root. Besides a tobacco powder basically obtained from the lamina of tobacco leaves, elements derived from the midrib or root of tobacco leaves may be contained in the tobacco filling.

There is no specific restriction on the particle size of the tobacco powder. In order for the product to fit well inside the oral cavity and feel better during use and for good release of the flavor component contained in the tobacco powder into the oral cavity, however, it is preferred that the tobacco powder be one that has passed through a 1.2-mm mesh. More preferably, the tobacco powder is one that has passed through a 1.0-mm mesh.

The tobacco species that serves as a raw material for the tobacco powder is not particularly limited. Examples include species within the Nicotiana genus, such as the flue-cured and burley cultivars of Nicotiana tabacum and the Brazilian cultivar of Nicotiana rustica. For the tobacco material and tobacco leaves, described later herein, too, the same species as these can be used.

Preferably, the tobacco powder is obtained as follows. First, at least one base is added to and mixed with a tobacco powder obtained by grinding tobacco leaves. The base that is added can be, for example, potassium carbonate and/or sodium carbonate, and it is preferred to add it as an aqueous solution. A pH-adjusting agent like potassium dihydrogenphosphate, furthermore, may be added, for example for the stabilization of nicotine during the manufacture of the oral pouch product. It is preferred to adjust the pH of the mixture after the addition of the base to 8.0 to 9.0.

An example of a percentage of the tobacco powder in this mixture is from 60 to 90 wt %.

After the addition of the base, heating is performed, for example for 0.5 to 3 hours, preferably 0.8 to 2 hours, for example under conditions under which the mixture temperature will be from 65°C to 90°C, preferably the mixture temperature will be from 70°C to 80°C. Through this, the sterilization of the tobacco powder is carried out.

The heating can be performed by one or both of heating by steam injection and heating with a jacket.

The pH of the mixture after heating is preferably from 8.0 to 9.0, and the water content of the mixture after heating is preferably from 10 to 50 wt %.

After the heating, the resulting processed tobacco powder is subjected to drying treatment by stopping the steam injection if necessary and performing heating with a jacket alone.

Cooling the powder at approximately 15°C to 25°C for approximately 1 hour thereafter will give the tobacco powder.

When a tobacco material containing a tobacco powder is used, the amount of tobacco material added is typically 0.001 wt % or more, preferably 0.01 wt % or more, more preferably 0.05 wt % or more in relation to the oral composition. For the savor of the oral composition, however, the amount of the tobacco material containing a tobacco powder added is typically 79 wt % or less, preferably 75 wt % or less, more preferably 70 wt % or less in relation to the oral composition. The amount of the tobacco material containing a tobacco powder added, furthermore, may be 45 wt % or less, may be 40 wt % or less, or may be 30 wt % or less in relation to the oral composition.

Alternatively, a nicotine-containing extract obtained through extraction from a nicotine-containing substance, such as tobacco leaves, may be contained as a nicotine source.

Of the forms described above, the addition of a nicotine carrier is particularly preferred from the viewpoint of appropriate supply of nicotine and the ease of handling. When a tobacco powder is added, furthermore, the color of the oral composition and the pouch product usually tends to be the color of the tobacco leaves, but when a colorless nicotine-containing compound is used, it is possible to provide a white composition and pouch product. For those users who like white pouch products, such a form is an advantage.

For the forms described above, one form may be applied alone, or two or more forms may be applied in combination.

The content of nicotine in the oral composition is not particularly restricted. For palatability to users, however, the content of nicotine is typically 0.001 wt % or more, preferably 0.01 wt % or more, more preferably 0.1 wt % or more. Additionally, the content of nicotine is typically 15.0 wt % or less, preferably 10.0 wt % or less, more preferably 5.0 wt % or less.

For this content of nicotine, the same ranges can be applied regardless of whether the nicotine source is, for example, the above-described substance including an ion-exchange resin and nicotine supported it, the tobacco material containing a tobacco powder, or the nicotine-containing extract.

It should be noted that when the nicotine is present as ions, the above content is a content as nicotine ions.

The content of nicotine in the oral composition can be measured by, for example, gas chromatography-mass spectrometer (GC-MS) or liquid chromatography (LC, UV detection).

### (Trehalose)

The oral composition in this embodiment contains trehalose. The trehalose can be, for example, α,α-trehalose or α,β-trehalose (also referred to as neotrehalose). α,α-trehalose is preferred because it is excellent in hygroscopicity.

Examples of forms of trehalose, furthermore, include crystalline hydrous trehalose and anhydrous trehalose. It is, however, preferred that the trehalose be anhydrous trehalose in particular. When the trehalose is anhydrous trehalose, its hygroscopic effect develops suitably. Examples of types of anhydrous trehalose include crystalline anhydrous trehalose and amorphous anhydrous trehalose. Amorphous anhydrous trehalose is more preferred because when being amorphous, anhydrous trehalose is expected to be highly hygroscopic by virtue of its large surface area.

The trehalose can be anhydrous trehalose or, alternatively, can be crystalline hydrous trehalose.

Anhydrous trehalose as mentioned in the present invention, furthermore, only needs to be substantially anhydrous trehalose, which is converted to crystalline hydrous trehalose and produces a strong dehydrating effect in that form. The anhydrous trehalose may be an anhydrous trehalose powder that contains the smallest possible amount of crystalline hydrous trehalose, typically less than 5%, desirably less than 1% as crystals. The coexistence of crystalline hydrous trehalose and anhydrous trehalose encourages, for example, the conversion of the anhydrous trehalose to crystalline hydrous trehalose and can lead to an improvement in the hygroscopic effect.

The content of the trehalose in the oral composition is from 21 to 99 wt % in relation to the entire oral composition. In order for the hygroscopic effect to be sufficiently strong, the lower limit is preferably 25 wt %, more preferably 30 wt %. For a product design that implements a savor of the oral product that its users like, however, the upper limit is preferably 80 wt %, more preferably 70 wt %, even more preferably 60 wt %, still more preferably 50 wt %.

The weight ratio of nicotine in relation to the trehalose contained in the oral composition, furthermore, is not particularly limited, but typically is 0.00001 or greater and 0.7 or less. In order for the hygroscopic effect of the trehalose to be sufficiently strong, the upper limit is preferably 0.5 or less, more preferably 0.1 or less, even more preferably 0.05 or less.

The form of the trehalose in the oral composition is not particularly limited; the trehalose may be present independently as a crystal powder within the composition or may be adhering to another raw material. When the composition is packaged using a packaging material like a pouch, furthermore, the trehalose may be in the form in which it has been infiltrated into that packaging material.

When the trehalose is present as a crystal powder, its average particle size is not particularly limited. From the viewpoint of manufacturability, however, it is preferred that the average particle size be from 100 to 1200 µm. The upper limit is more preferably 1000 µm, even more preferably 500 µm, particularly preferably 150 µm. When the average particle size is in these ranges, it is easy to pack the desired amount, for example into pouches, and it is unlikely that dust is produced. Feel on the tongue and texture in the mouth, furthermore, are likely to be good.

A crystal powder of trehalose that falls within these ranges can be obtained by, for example, passing trehalose through a mesh having a particular pore size specified in JIS Z8801-1 (2006). An average particle size herein is measured by dry sieving (JIS Z 8815-1994). An average particle size herein, furthermore, refers to the particle size at which the cumulative volume is 50% in a particle size distribution (D50), unless specifically stated.

An example of a method for attaching the trehalose to another raw material is the method of dissolving the trehalose in a solvent, such as water, adding the solution to or spraying the solution onto each raw material for the composition or the entire composition, and optionally drying the raw materials or composition.

### (Substrate)

Trehalose can be used as a substrate in known oral compositions. The oral composition in this embodiment, therefore, exhibits good formability even without a substrate other than trehalose. The oral composition, however, may further contain at least one substrate other than trehalose. The type of substrate is not particularly restricted; substances such as polysaccharides and porous structures capable of adsorbing and holding water can be employed. Specifically, the substrate is preferably one or more selected from the group consisting of cellulose, microcrystalline cellulose (MCC), spherical cellulose, and porous cellulose and more preferably is cellulose for flexibility in the adjustment of the bulk density of the oral composition and because it displays a white color. One of such substances may be used alone, or any two or more types may be used in combination in any proportions.

The percentage of the substrate other than trehalose in the oral composition (when the composition contains two or more substrates, their total percentage) is not particularly restricted. For the quality improvement of reduced exudation of water during manufacture or product storage and in order that an appearance desirable to users will be imparted through the enhancement of the whiteness of the product, however, the percentage may be 0 wt % or more. Preferably, the percentage is 5 wt % or more, more preferably 10 wt % or more. There is, furthermore, no need to particularly restrict the upper limit, but in view of the limits on how much the other raw materials can be contained, the percentage is typically 70 wt % or less.

Preferably, the percentage is 68 wt % or less, more preferably 65 wt % or less.

### (Water)

The percentage of water in (water content of) the oral composition is typically 0.9 wt % or more for the ease of manufacture of the oral composition. From viewpoints such as improved manufacturing efficiency, improved anticaking properties, and reduced stickiness of the oral composition, furthermore, it is preferred that the percentage of water be 30 wt % or more, more preferably 45 wt % or more. Moreover, the water content is typically 60 wt % or less, preferably 50 wt % or less. Additionally, the percentage of water may be 40 wt % or less, may be 30 wt % or less, or may be 20 wt % or less. This percentage of water can be adjusted by adjusting the quantity of water added and providing heating treatment or drying treatment during the manufacturing stage.

The percentage of water in the oral composition can be adjusted according to the type of product (moist or dry) as needed. For the moist type, for example, the percentage of water is typically 10 wt % or more, preferably 20 wt % or more, more preferably 30 wt % or more, and is typically 60 wt % or less, preferably 50 wt % or less. By contrast, for the dry type, the percentage of water is typically 0.9 wt % or more and 20 wt % or less, preferably 10 wt % or more and 15 wt % or less.

The weight ratio of water in relation to the trehalose contained in the oral composition, furthermore, is not particularly limited, but typically is 0.01 or greater and 2 or less. In order for the hygroscopic effect of the trehalose to be sufficiently strong, the upper limit is preferably 1 or less, more preferably 0.5 or less, even more preferably 0.1 or less, particularly preferably 0.05 or less.

The percentage of water in (water content of) the oral composition is measured using a heat-drying moisture analyzer (e.g., HB 43-S: manufactured by METTER TOLEDO). For the measurement, a sample is put into a predetermined container and heated to an attained temperature of 100°C. The measurement is concluded at the time when the amount of change becomes 1 mg or less per 60 seconds, and the water content is calculated from the measured weights before and after the heating.

For the moist type, there can be the additional advantage of the prevention of the separation of water from the product as a result of the trehalose absorbing and holding water.

### (Extra Substances)

Besides nicotine, trehalose, a substrate or substrates, and water, the oral composition may contain extra substances. Examples of extra substances include flavoring agents, pH-adjusting agents, sweeteners, humectants, bitterness reducers, white coloring agents, emulsifiers, release agents, and gelling agents.

The percentages of extra substances in the oral composition are not particularly restricted. For substances without a description of preferred percentages, their amounts can be adjusted according to the product design as needed.

The type of flavoring agent is not particularly restricted, and examples include menthol, leaf tobacco extract, natural vegetable flavoring agents (e.g., cinnamon, sage, herbs, chamomile, kudzu, sweet hydrangea leaves, clove, lavender, cardamom, caryophyllus, nutmeg, bergamot, geranium, honey essence, rose oil, lemon, orange, cinnamon bark, caraway, jasmine, ginger, coriander, vanilla extract, spearmint, peppermint, cassia, coffee, celery, cascarilla, sandalwood, cocoa, ylang-ylang, fennel, anise, licorice, St. John's bread, plum extract, and peach extract), saccharides (e.g., glucose, fructose, isomerized sugar, caramel, honey, and molasses), types of cocoa (powder, extract, etc.), esters (e.g., isoamyl acetate, linalyl acetate, isoamyl propionate, and linalyl butyrate), ketones (e.g., menthone, ionone, damascenone, and ethyl maltol), alcohols (e.g., geraniol, linalool, anethole, and eugenol), aldehydes (e.g., vanillin, benzaldehyde, and anisaldehyde), lactones (e.g., γ-undecalactone and γ-nonalactone), animal flavoring agents (e.g., musk, ambergris, civet, and castoreum), and hydrocarbons (e.g., limonene and pinene). One of such substances may be used alone, or any two or more types may be used in combination in any proportions.

The type of pH-adjusting agent is not particularly restricted, and examples include sodium carbonate, sodium hydrogencarbonate, potassium carbonate, potassium hydrogencarbonate, anhydrous sodium phosphate, sodium dihydrogenphosphate, and sodium citrate. From the viewpoint of impact on the taste that the product exhibits, sodium carbonate, potassium carbonate, and sodium dihydrogenphosphate are preferred. One of such substances may be used alone, or any two or more types may be used in combination in any proportions.

As for sweeteners (excluding trehalose), examples include sugar alcohols, such as xylitol, maltitol, and erythritol, and sweeteners such as acesulfame potassium, sucralose, and aspartame. Sugar alcohols are preferred from the viewpoint of taste adjustment. One of such substances may be used alone, or any two or more types may be used in combination in any proportions.

Bitterness reducers are not particularly restricted, and an example is soybean lecithin. Soybean lecithin is phospholipids, with examples including phosphatidylcholine, phosphatidylethanolamine, and phosphatidic acid. One of such substances may be used alone, or any two or more types may be used in combination in any proportions.

The type of humectant is not particularly restricted, and examples include glycerine and propylene glycol. From the viewpoint of product storability, glycerine is preferred. One of such substances may be used alone, or any two or more types may be used in combination in any proportions.

The type of white coloring agent is not particularly restricted, and examples include particulate silicon dioxide, titanium dioxide, and calcium carbonate. From the viewpoint of the impact of taste on the product, particulate silicon dioxide is preferred. One of such substances may be used alone, or any two or more types may be used in combination in any proportions.

The type of emulsifier is not particularly restricted, and examples include emulsifiers that are added to foods. An example is one or more emulsifiers selected from the group consisting of sucrose fatty acid esters, organic acid glycerine fatty acid esters, and polyglycerine fatty acid esters and lecithins. Examples of sucrose fatty acid esters include sucrose palmitate and sucrose stearate. Examples of organic acid glycerine fatty acid esters include succinic acid glycerine fatty acid esters and diacetyltartaric acid glycerine fatty acid esters. Examples of polyglycerine fatty acid esters include decaglycerine fatty acid esters.

For the content of the emulsifier or emulsifiers in the oral composition, a typical example is 1 wt % or more and 20 wt % or less, and a preferred example is 5 wt % or more and 15 wt % or less.

Release agents mitigate the trouble of the adhesion of the oral composition to manufacturing equipment, such as a mixer and a kneader, during the manufacture of the oral composition, helping improve manufacturing efficiency. Release agents, furthermore, reduce caking (the aggregation and solidification of the materials constituting the oral composition) by decreasing the adhesiveness between the materials and help reduce the stickiness of the oral composition as well. As a result, characteristics of products made with the oral composition are improved; for example, the appearance, feel during use, such as feel in the mouth, flavor, etc., of the products made with the oral composition improve.

The type of release agent is not particularly restricted as long as the above advantages are delivered. Examples include compounds such as silicon dioxide, magnesium oxide, calcium silicate, magnesium silicate, calcium phosphate, calcium stearate, and magnesium stearate. Of these, it is preferred that the release agent be silicon dioxide because it is superior in the advantages described above and has little impact on flavor. One release agent may be used alone, or any two or more types may be used in combination in any proportions.

The percentage of the release agent in the oral composition (when the composition contains two or more release agents, their total percentage) is not particularly restricted. In order to ensure a good flavor, however, the percentage is typically 0.05 wt % or more. Preferably, the percentage is 0.1 wt % or more, more preferably 0.5 wt % or more. The percentage, furthermore, is typically 3.0 wt % or less, preferably 2.5 wt % or less, more preferably 2.0 wt % or less.

Examples of gelling agents include glucomannan, galactomannan, carrageenan, pectin, gum arabic, xanthan, gellan, gum tragacanth, and alginic acid. One of such substances may be used alone, or any two or more types may be used in combination in any proportions.

The percentages of the ingredients described above (excluding the percentage of water) can also be calculated from the amounts of raw materials initially added.

### (Form of the Oral Composition)

The oral composition in this embodiment can be made in any form, such as a solid like a powder, granules, or fine particles or a semi-solid like gel or paste.

The composition, furthermore, can be applied to a known oral product form. The product may be in any form that is orally administered, including dissolving tablets, disintegrating tablets, a powder, granules, gel, paste, chewing gum, patches, capsules, sublingual tablets, troches, pouches, hard candies, and mucoadhesive sheets/films.

### (Particle Size of the Components of the Oral Composition)

When the oral composition is a solid like a powder, granules, or fine particles, the size of the granular pieces is not particularly restricted. For example, it is preferred that the components of the oral composition after drying be ones that meet the classification conditions described below.

The dried oral composition is preferably one that has been classified with a sieve having a sieve aperture specified below. To ensure good feel in the mouth during use by the user and for the ease of handling during manufacturing and controlled variations in quality, the dried oral composition is typically one that passes through a sieve having a sieve aperture of 15 mm (< 15 mm). Preferably, the dried oral composition is one that passes through a sieve having a sieve aperture of 10 mm (< 10 mm), more preferably one that passes through a sieve having a sieve aperture of 5 mm (< 5 mm), even more preferably one that passes through a sieve having a sieve aperture of 3.2 mm (< 3.2 mm). For example, when all of the oral composition dried to a water content of 5 wt % or less has passed through a sieve with a sieve aperture of 3.2 mm, it indicates that the maximum particle size after drying of the components of the oral composition is 3.2 mm or less.

There is no need to set the lower limit to the particle size of the components of the oral composition after drying, but typically, the particle size is 3 µm or more. When the oral composition is used for an oral pouch product as described later herein, a particle size of 3 µm or more facilitates the prevention of leakage from the pouch.

The method for preparing the dried oral composition described above is not particularly restricted as long as it allows for the reduction of the water content of the oral composition to 5 wt % or less. An example is the method of preparing it by allowing the oral composition to stand for a predetermined duration under temperature conditions such as normal temperature or 70°C to 80°C.

The maximum particle size of the components of the oral composition can be increased/decreased as needed, for example by adjusting the particle size, water content, etc., of the ion-exchange resin with nicotine supported on it.

### (pH of the Oral Composition)

The pH of the oral composition at a measuring temperature of 25°C is not particularly restricted, but typically is 7.0 or higher because of impact on the taste of the product. Preferably, the pH is 7.5 or higher, more preferably 8.0 or higher The pH, furthermore, is typically 10.0 or lower, preferably 9.5 or lower, more preferably 9.0 or lower. This pH can be adjusted by controlling the amount of pH-adjusting agent added. It should be noted that any pH value herein, not only the above pH values, is a value measured at a measuring temperature of 25°C.

This pH of the oral composition at a measuring temperature of 25°C can be measured using a pH analyzer (e.g., LAQUA F-72 with a flat ISFET pH electrode: manufactured by HORIBA, Ltd.), by adding 20 ml of water to 2 g of the oral composition, shaking the mixture for 10 minutes, and analyzing the supernatant.

The calibration of the equipment is performed by, for example, three-point calibration using a phthalate pH standard solution (pH 4.01), a phosphate pH standard equimolal solution (pH 6.86), and a tetraborate pH standard solution (pH 9.18) (all from Wako Pure Chemical Industries, Ltd.).

### (Method for Manufacturing the Oral Composition)

The method for manufacturing the oral composition is not particularly restricted as long as it allows for the manufacture of the oral composition described above through the mixing of at least nicotine and trehalose. For example, by putting all raw materials into a mixer and mixing them together, the oral composition can be manufactured. A preferred form of the composition manufacturing process, however, is as presented below. It should be noted that the raw materials in the composition manufacturing process can be the ingredients described earlier herein.

First, nicotine and optionally water and extra substances are mixed together to give a first mixture. During this, heating may optionally be performed. The order of mixing of the raw materials, furthermore, is not particularly restricted; the materials may be put into the mixer and mixed together in any order or simultaneously. When solid raw materials and liquid raw materials are used, it is particularly preferred to mix the solid raw materials until uniform, then add the liquid raw materials, and further mix the materials.

Additionally, the resulting first mixture and an emulsifier may be mixed together to give a second mixture by spraying a solution of the emulsifier onto the first mixture while stirring the first mixture. Because the emulsifier will produce an anticaking effect, caking will be less likely to occur in subsequent steps. The solvent for the emulsifier is not particularly restricted as long as it allows the emulsifier to be dissolved in it, but preferably is an alcohol solvent, such as ethanol. To prevent much of the solvent for the emulsifier from remaining in the second mixture, heating treatment may be applied during the spraying of the solution of the emulsifier onto the first mixture or after the end of the spraying.

A treatment in which the first or second mixture is dried, furthermore, may be performed (drying step). After that, a treatment in which the mixture is cooled may be performed. The cooling may be natural cooling or may be performed using some cooling means (cooling step). The drying method is not particularly limited, but examples include vacuum drying and hot-air drying. By adjusting the pressure, temperature, and duration as needed during drying, the water content of the first or second mixture can be adjusted, for example to a desired value within the range of 5 to 60 wt %. This facilitates the adjustment of the water content of the oral composition as the product of interest.

To the mixture obtained in the above steps (or the drying step and/or cooling step), an aqueous solution containing a pH-adjusting agent, a sweetener, such as acesulfame potassium, a flavoring agent, such as menthol, a bitterness reducer, such as soybean lecithin, and a humectant, such as glycerine, are optionally added (additive addition step). The resulting mixture is shaped (shaping step) to give the desired oral composition.

When materials such as the above additives are added, the materials may be solid or may be added as aqueous solutions, in which the materials are dissolved in water. When the materials are added as aqueous solutions, they may be pre-dissolved in a predetermined amount of water before addition so that the final water content of the oral pouch product will be achieved.

The trehalose can be added in any of the steps described above. When the trehalose is attached to another raw material, the trehalose can be attached before the addition of that raw material. It is also possible to attach the trehalose to the entire composition after the shaping step described above. Meanwhile, an example of the most preferred approach is to add the trehalose immediately before the shaping step and mix the materials together. By adding the trehalose immediately before the shaping step, the contact between the trehalose and water during the course of manufacture can be minimized.

### [Oral Pouch Product]

Another embodiment of the present invention is an oral pouch product composed of the oral composition described above and a packaging material in which the oral composition is packaged (hereinafter occasionally referred to as a "pouch"). During the use of the oral pouch product, saliva present in the oral cavity permeates through the pouch and dissolves ingredients in the composition included in the pouch, and then the dissolved ingredients are transported out into the oral cavity, passing through the pouch. The ingredients in the composition transported out into the oral cavity can be absorbed through the mucosa.

In the oral pouch product, the oral composition is preferably a solid like a powder, granules, or fine particles. The particle size of the oral composition in this case is as described above.

### (Packaging Material)

The packaging material (pouch) is not particularly restricted and can be a known material as long as it is one in which the oral composition can be packaged and that is insoluble in water and, at the same time, can have permeability to liquids (such as water and saliva) and water-soluble ingredients in the composition. An example of a material for the pouch is cellulose-based nonwoven fabric, and commercially available nonwoven fabric may also be used. The pouch product can be produced by shaping a sheet made from such a material into a bag, putting the composition described above into the bag, and sealing the bag by means such as heat sealing.

The grammage of the sheet is not particularly restricted and typically is 12 gsm or more and 54 gsm or less, preferably 24 gsm or more and 30 gsm or less.

The thickness of the sheet is not particularly restricted and typically is 100 µm or more and 300 µm or less, preferably 175 µm or more and 215 µm or less.

At least one of the inner surface or outer surface of the pouch may be partially coated with a water-repellent material. For use as the water-repellent material, a water-repellent fluoropolymer is suitable. Specifically, an example of a water-repellent fluoropolymer of this type is AsahiGuard^{®}, manufactured by Asahi Glass Co., Ltd. Water-repellent fluoropolymers are materials that are used to coat packaging materials for fat-containing foods and products, including confectionery, dairy products, ready-prepared foods, fast food, and pet food. Water-repellent fluoropolymers of this type, therefore, are safe even when applied to a pouch intended to be placed in the oral cavity. It should be noted that this water-repellent material is not limited to fluoropolymers; it can be any material that has a water-repellent effect, such as a paraffin resin, silicone resin, or epoxy resin.

The pouch may contain any ingredients, with examples including raw materials for adjusting flavor and taste, flavoring agents, additives, tobacco extract, and colorants. The pouch, furthermore, may contain trehalose. The form in which these ingredients are contained is not particularly restricted, and examples include the form in which the ingredients are applied to the surface of the pouch or infiltrated into the pouch and, when the pouch is made of fibers, the form in which the ingredients are introduced into the fibers.

The appearance of the pouch, moreover, is not particularly restricted either. The pouch may not only be a nontransparent one but also a translucent or transparent one. In the latter case, the composition packaged in the pouch is visible through the pouch.

The size and weight of the oral pouch product are not particularly restricted. For the size of the oral pouch product before use, the longer sides may measure 25 mm or more and 40 mm or less or may measure 28 mm or more and 38 mm or less, and the shorter sides may measure 10 mm or more and 20 mm or less or may measure 14 mm or more and 18 mm or less. The weight of the oral pouch product before use, furthermore, may be 0.1 g or more and 2.0 g or less or may be 0.3 g or more and 1.0 g or less.

The percentage of the weight of the oral composition to the total weight of the oral pouch product is not particularly restricted, but typically is 80 wt % or more. Preferably, the percentage is 85 wt % or more, more preferably 90 wt % or more. The percentage, furthermore, is typically 99 wt % or less, preferably 97 wt % or less, more preferably 95 wt % or less.

### <Method for Manufacturing the Oral Pouch Product>

A method for manufacturing the oral pouch product according to this embodiment includes a packaging step in which the oral pouch product is produced by packaging the oral composition according to an embodiment of the present invention in a packaging material.

The oral composition is packaged in a packaging material to give the oral pouch product (packaging step). The method for packaging the composition is not particularly restricted, and known methods can be applied. For example, known methods such as the method of putting the oral composition described above into a bag-shaped nonwoven fabric and then sealing the bag can be used.

### <Applications of the Oral Pouch Product>

The applications (forms of use) of the oral pouch product are not particularly restricted, but examples include oral tobacco, such as chewing tobacco, snuff, and compressed tobacco, or nicotine-containing preparations referred to as nicotine pouches. Nicotine pouches are products intended for users to put between their lip and gum in the oral cavity, allowing them to savor the taste and flavor.

### [Chewing Gum]

Another embodiment of the present invention is chewing gum made from the oral composition described above. It should be noted that "chewing gum" encompasses so-called bubblegum. As a result of the user's chewing the chewing gum, the ingredients contained in the composition mix with saliva and can be absorbed through the oral mucosa.

In general, chewing gum includes at least one water-soluble material (a sweetener, an acidulant, an antioxidant, and/or a flavoring agent) and a gum base portion that is water-insoluble and chewable (an elastomer, a plasticizer, a filler (e.g., a binder), and a softening agent (e.g., fat, wax, or an emulsifier)).

The chewing gum may be in any form and any shape. For example, it may be in a form selected from pellets, sheets, tablets, troches, cubes, pillows, disks, and balls. The chewing gum may have been shaped by extrusion, compression, or rolling.

The chewing gum, furthermore, may have its surface coated with a coating material. The coating material can be one used to cover articles such as medicinal products and confectionery, and a rigid coating, film coating, or flexible coating with any composition can be used.

### [Lozenge]

Another embodiment of the present invention is a lozenge made from the oral composition described above. As used herein, the term "lozenge" refers to a preparation for oral administration that is applied to, for example, the oral cavity or pharynx by slowly dissolving or disintegrating it inside the oral cavity and includes preparations such as an "orally dissolving tablet," an "orally disintegrating tablet," and a "hard candy." A lozenge can be manufactured as a sublingual tablet, which is intended to be placed under the tongue, a troche, which is intended to dissolve on the tongue, a chewable tablet, or a drop or other types of tablets. By orally administering the lozenge to the user, it is possible to cause ingredients contained in the composition to be absorbed at the oral mucosa or pharynx.

In this embodiment, the oral composition can be in any form, such as a solid like a powder, granules, or fine particles or a vitreous solid obtained by melting the composition into its liquid form once and then solidifying the liquid.

The lozenge, furthermore, may have its surface coated with a coating material. The coating material can be one used to coat articles such as medicinal products and confectionery, and a rigid coating, film coating, or flexible coating with any composition can be used.

### [Mucoadhesive Film]

Another embodiment of the present invention is a mucoadhesive film made from the oral composition described above. A mucoadhesive film typically has, as a substrate, a matrix whose base component is a hydrophilic, water-soluble polymer, and is maintained in its dry state during storage. A mucoadhesive film absorbs water, for example in saliva, when placed in the oral cavity of the user, and is used by attaching it to the oral mucosa. The film itself may completely dissolve or disintegrate because of the water, for example in saliva.

The hydrophilic, water-soluble polymer can be a known one that can be used in products such as foods, and examples include cellulose, polyvinyl alcohol, polyvinyl acetate, polyvinyl pyrrolidone, acrylic acid polymers, methacrylic acid polymers, pullulan, guar gum, xanthan gum, carrageenan, gum arabic, gelatin, and starch, along with their salts, derivatives, and copolymers. These can be used individually or in combination.

The ingredients contained in the composition are released as a result of dissolution or disintegration caused by the action of saliva and can be absorbed by the oral mucosa.

### EXAMPLES

The present invention will now be described in further detail with examples. The present invention, however, is not limited to the descriptions in the following examples unless it departs from the scope thereof.

Raw materials used in the Examples are presented below.
- Nicotine polacrilex: Nicotine Polacrilex 20% manufactured by Contraf nicotex
- Pectin: Classic CU 902 manufactured by H&F

It should be noted that the nicotine polacrilex contains 20 wt % nicotine.

Oral compositions of dry type were prepared by mixing raw materials until uniform according to the weight proportions indicated in Table 1.

### [Table 1]

**Table 1**

| Raw materials (wt%) | Example 1 | Example 2 | Example 3 | Example 4 |
|---|---|---|---|---|
| Nicotine polacrilex | 5.0 | 5.0 | 5.0 | - |
| Purified nicotine | - | - | - | 1.0 |
| Pectin | 2.0 | 2.0 | 2.0 | - |
| α,α-trehalose | 22.0 | 36.3 | 41.2 | 99.0 |
| Microcrystalline cellulose | 51.4 | 37.1 | 32.2 | - |
| Na₂CO₃ | 3.4 | 3.4 | 3.4 | - |
| Glycerol | 11.2 | 11.2 | 11.2 | - |
| Flavoring agent | 4.9 | 4.9 | 4.9 | - |
| Total | 100.0 | 100.0 | 100.0 | 100.0 |

Oral compositions of moist type were prepared by mixing raw materials until uniform according to the weight proportions indicated in Table 2.

### [Table 2]

**Table 2**

| Raw materials (wt%) | Example 5 | Example 6 | Example 7 | Example 8 | Example 9 | Example 10 |
|---|---|---|---|---|---|---|
| Purified nicotine | 1.0 | 1.0 | 1.0 | 1.0 | 1.0 | 1.0 |
| Powdered tobacco | 0.01 | 0.01 | 0.01 | 0.01 | 0.01 | 0.01 |
| Cellulose | - | 14.64 | - | 14.64 | 14.64 | 14.64 |
| α,α-trehalose | 25.4 | 25.4 | 40.3 | 40.3 | 50.3 | 68.2 |
| Gellan gum | 0.32 | 0.32 | 0.32 | 0.32 | 0.32 | - |
| Glycerine monoester | 0.76 | 0.76 | 0.76 | 0.76 | 0.76 | - |
| Calcium lactate | 0.76 | 0.76 | 0.76 | 0.76 | 0.76 | - |
| NaCl | 2.73 | 2.73 | 2.73 | 2.73 | 2.73 | - |
| K₂CO₃ | 7.64 | 7.64 | 7.64 | 7.64 | 7.64 | - |
| Acesulfame K | 0.01 | 0.01 | 0.01 | 0.01 | 0.01 | 0.01 |
| Flavoring agent | 1.46 | 1.46 | 1.46 | 1.46 | 1.46 | 1.46 |
| Water | 60.0 | 45.0 | 45.0 | 30.0 | 20.0 | 15.0 |
| Total | 100 | 100 | 100 | 100 | 100 | 100 |

Examples 1 to 10 above can be made into oral pouch products, for example by putting them into a packaging material, such as nonwoven fabric, and sealing the packaging material.

## Claims

1. An oral composition comprising nicotine and trehalose, wherein:
a content of the trehalose is from 21 to 99 wt %.

2. The oral composition according to claim 1, wherein the trehalose is α,α-trehalose or α,β-trehalose.

3. The oral composition according to claim 1 or 2, wherein the trehalose is α,α-trehalose

4. The oral composition according to any one of claims 1 to 3, wherein the trehalose is anhydrous trehalose.

5. The oral composition according to any one of claims 1 to 4, wherein the trehalose is present as a crystal powder.

6. The oral composition according to claim 5, wherein an average particle size of the trehalose is from 100 to 1200 µm.

7. An oral pouch product comprising the oral composition according to any one of claims 1 to 6 and a packaging material in which the oral composition is packaged.
